# EUROPEAN PATENT SPECIFICATION

(11) **EP 3 720 364 B1**
(45) Date of publication and mention of the grant of the patent: **09.02.2022**
(21) Application number: 18826780.1
(22) Date of filing: 07.12.2018
(51) Int. Cl.: A61B 17/04, A61B 17/062, A61B 17/06

(54) **A SEWING DEVICE**
NÄHVORRICHTUNG
SYSTÈME DE SUTURE

(30) Priority: 08.12.2017 GB 201720496
(43) Date of publication of application: 14.10.2020
(73) Proprietor: IP2IPO Innovations Limited, London EC4N 8AF (GB)
(72) Inventor: YANG, Guang-Zhong, London SW7 2AZ (GB); SENECI, Carlo Alberto, London SW7 2AZ (GB); HU, Yang, London SW7 2AZ (GB)
(74) Representative: Potter Clarkson
(86) International application number: PCT/GB2018/053562
(87) International publication number: WO 2019/111017

(56) References cited:
- WO-A2-2008/045333
- DE-C1- 4 124 383
- US-A- 5 690 652
- US-A1- 2004 193 146
- US-A1- 2015 351 748
- US-A1- 2016 066 902

## Description

This invention relates to a sewing device, and particularly, but not exclusively to a sewing device suitable for use in medical applications, and particularly to a suturing device for use in minimal invasive surgery.

Within this specification the terms sew and sewing will be used interchangeably with the words suture and suturing.

The act of suturing during minimal invasive surgery can be difficult. The process requires coordinated manipulation with both hands of a small needle, often a curved needle, and using two grippers (needle drivers).

Under the constraints of a surgical port, performing a tissue stitching task is difficult because a straight shaft gripper lacks the dexterity to drive the needle around its geometrical centre.

On the other hand, knot tying is a key part in introcorporal suturing and can be very time consuming, especially when performing endoluminally in a narrow workspace.

The task of tying a knot generally requires using one instrument to wind the suturing thread onto the other instrument. It is very easy for the thread loop to come off from the wound instrument. For this reason, precise coordination between the two instruments is required.

To mitigate the difficulties in introcorporal suturing, and to speed up the procedure, a variety of suturing devices have been developed to assist introcorporal suturing. Many of these devices are manually operated.

US 2014/0257345 describes a surgical suture apparatus for open and/or endoscopic surgery. The apparatus is configured to pass a short, straight, double ended surgical needle backwards and forwards between a first jaw element and a second jaw element. The jaw elements each include a holding device to hold a respective needle end of the surgical needle.

US 5,480,406 describes a method for using an endoscopic suturing apparatus to place various knots in suture. The described apparatus comprises a nearly straight needle which may be passed between two jaws.

A disadvantage of a device using a short straight needle is that it can be difficult to pierce through thick layers of tissue, without applying a large pressure to the tissue.

US 2015/0057683 discloses an apparatus and method for minimally invasive suturing. The disclosed suturing device comprises, *inter alia,* a suturing head assembly and a suturing needle having a pointed end and a blunt end. The suturing needle is capable of rotating about an axis approximately perpendicular to a longitudinal axis of the proximal section of the device. The pointed end of the suturing needle is positioned within the suturing head assembly prior to and after rotation of the suturing needle.

To perform suturing, an opening area is created on the circular housing so that when the tissue appears in the opening area, it can be pierced by the rotating needle. To drive the needle, notches are created on the needle body and a spring-loaded pin is used for engaging and rotating the suturing needle.

One drawback of this suturing device is that the needle is rotated within a circular housing which has a limited opening area only in which to interact with the tissue. Therefore it cannot create relatively large stitches. Furthermore the needle needs to rotate a full circle by engaging and disengaging twice with the spring-loaded spring to perform a single stitch.

Another problem with prior art devices is that in general, the needle is notched. The notch is used to drive the needle in the housing of the device.

WO 2008/045333 discloses a handle assembly for operating a surgical instrument which includes a housing; a trigger operatively supported on the housing; and at least one actuation cable operatively associated with the trigger and extending from the housing in such a manner that an actuation of the trigger imparts both an axial translation of the actuation cable and a rotation to the actuation cable.

US 5,690,652 discloses an operating mechanism which carries at its free end a suturing head comprising two jaws between which a needle with opposite needle tips and a central eye can be exchanged while being alternately locked with the two jaws, the whole procedure being controllable by a single handle.

DE 41 24 383 discloses a suture applicator comprising a head composed of two parts. The first one is rigid and parallel to the instrument axis, while the second one pivots round an axis orthogonal to the first axis for the jaw closure and opening. A recessed and pointed clamp at the leading end of the first jaw has its axis normal to that of the instrument and contains a needle , which is adjusted by a slider, moving along the instrument axis and is clamped at one end. The other free end of the needle is held in a clamp recessed into the pivotal jaw and held in place by springs gripping round the needle bead. When the jaws are shut, i.e. without clamping the needle, ribbed surfaces on the two jaws engage so that the needle and thread lie concealed between them.

US 2015/351748 discloses a surgical suturing device comprising an arced needle having a leading end, a trailing end, and a length of suture. A needle driver is operable to drive the needle in a first rotational direction along a circular path and a cleat projects into the circular path and is operable to engage and prevent the needle from rotating in a second rotational direction opposite the first rotational direction.

According to the present invention there is provided a sewing instrument comprising: a proximal end and a distal end; a longitudinal axis; a sewing device being positioned at the distal end of the sewing instrument, which sewing device comprises:
a curved needle having a first end and a second end, a first point at the first end and a second point at the second end, the needle further comprising a thread receiving portion;
a first jaw and a second jaw, which jaws are moveable between an open position and a closed position, the first jaw being adapted to releasably hold the first end of the needle, and the second jaw being adapted to releasably hold the second end of the needle; and
a lock mechanism comprising a first lock for locking the needle in the first jaw, and a second lock for locking the needle in the second jaw;
the sewing instrument further comprising: a first drive mechanism for moving the jaws between the open position and the closed position; and a second drive mechanism for operating the first and second locks wherein the second drive mechanism comprises a first lock tendon attachable to the first lock, and a second lock tendon attachable to the second lock.

The sewing instrument, and particularly the sewing device according to embodiments of the invention may be used in a wide range of applications, both medical and non-medical.

The sewing device according to embodiments of the invention may be used in minimally invasive procedures. Such procedures may include suturing and knot tying in laparoscopic scenarios which may be either manual or robotic. Other minimally invasive procedures may include suturing and knot tying in flexible endoscopic scenarios either manual or robotic.

The sewing device according to embodiments of the invention may also be used in external medical procedures which may include the suturing of skin wounds. This procedure could potentially be automated by using a software to track the wound shape and by having a robot holding the probe.

Other medical applications involve the use of textiles. For example, the manufacture of a stent graft that consists essentially of a sleeve may be facilitated through use of the sewing device according to embodiments of the invention. Such a stent graph may be produced in a customised way through use of the sewing device according to embodiments of the invention. This ensures that each patient's needs are taken into account. In addition the process may be faster, more precise and it may generate more complex designs than those that are used commonly in stent grafts in the prior art.

The sewing device according to embodiments of the invention also has application in non-medical textile applications. This is because the sewing device essentially comprises a miniature sewing machine. Due to the shape of the sewing device according to embodiments of the invention it is possible to use the device to sew sleeves and tubes from the inside. It is not therefore required to flip the sleeves/tubes inside out for sewing. In other words, all of the sewing can be carried out on a single side of a piece of textile.

This in turn means that the sewing device according to embodiments of the invention is adapted to produce longer sleeves than may be the case when traditional sewing/suturing machines are used since flipping a long and thin tube is not easily achievable.

Fabric made sleeves and tubes have a wide range of applications including clothing, electrical cables, water pipes electrical cable protection, water pipe protection, for example.

For certain applications a plurality of sewing devices according to embodiments of the invention may be used to allow for the simultaneous stitching of different threads. This could allow the production of smart clothing, for example, the sewing devices may be used to insert sensors into clothing.

Through use of a plurality of sewing devices according to embodiments of the invention, rather than one multi-material large sewing device, for example, more flexibility is achieved.

The sewing device according to embodiments of the invention could also be used as a hand held sewing machine. Such a hand held sewing machine would be useful to dress makers when making clothing repairs particularly in scenarios such as in theatre where clothes may be ripped during shows. The sewing device according to embodiments of the invention could also be used for many textile applications that feature single sided stitching. Single sided stitching is used to manufacture a range of products including sportswear and automotive items. In particular the sewing device according to embodiments of the invention could be used to produce covers for steering wheels and car seats for example.

The invention will be described mainly with reference to a medical application. However it is to be understood that, as explained above, the invention has a wider application in non-medical areas.

The needle may have any suitable curve, but in some embodiments of the invention the needle defines an arc of a circle. The arc may extend over 160 to 200°.

The first and second jaws may be configured to open and close symmetrically with respect to a longitudinal axis of the sewing device.

The jaws may be moveable by any convenient means, but in some embodiments of the invention each jaw is rotatable between the open position and the closed position.

In some embodiments of the invention the first and second jaws are each pivotable about a common pivot pin.

In such embodiments of the invention the the pivot pin may be positioned substantially at the centre of a circle, an arc of which is defined by the needle. In such embodiments of the invention the needle is adapted to move around the circumference of this circle and the first and second jaws are adapted to pivot about the centre of the circle.

In such embodiments of the invention, the first and second jaws will follow a trajectory that extends around the circumference of this circle when moving between the open position and the closed position.

When the first and second jaws move towards the closed position, the ends of the jaws remote from the pivot pin converge towards one another.

The thread receiving portion formed in the needle allows a thread to be attached to the needle. This means that movement of the needle will result in the thread being drawn through the tissue through which the needle is drawn.

When one jaw holds an end of the needle to drive it to pierce tissue, the exposed point is driven along its tangential direction.

When a point of the needle emerges from tissue, it is driven into the needle receiving portion of the other jaw. At this point the locking mechanism unlocks the needle receiving portion of the first jaw, and the lock mechanism causes the second jaw to lock and hold the needle in position. The thread is then drawn through the tissue, and thread is left inside the tissue and one stitch is finished.

The needle may then be passed back to the first jaw to repeat the stitching process until the wound is completely closed.

Because the first and second jaws are moveable between a first position and a second position, when the sewing device is inserted into a surgical port in order to be inserted into the body of a patient, the first and second jaws may be placed in the closed position with the needle locked to one jaw. This ensures that the needle is not exposed during insertion of the sewing device into a patient. Such insertion usually occurs through a relatively small trocar port. After insertion, the first and second jaws are caused to move to the open position in order to perform the stitching.

Because the first and second jaws are adapted to releasably hold a respective end of the needle of the sewing device, movement of the jaws to the open position can cause an end of the needle to be exposed. Because the needle has a point at either end, when one of the ends of the needle is exposed, it is ready to be inserted into, for example tissue of a patient in order to begin the suturing process.

Once an end of the needle has passed through tissue of a patient, that end may then be held by a respective jaw, and the other end of the needle may be released in order that the needle and thread may be pulled through the tissue of a patient. This movement allows a thread attached to the needle to be pulled through the tissue as part of the suturing process.

Tissue damage when the needle is inserted into a patient's tissue is reduced through use of a curved needle. In addition, conventional needles used in surgery tend to be curved, and therefore by using a curved needle, the suturing device of embodiments of the invention conforms with conventional surgical suturing practices.

In embodiments of the invention each jaw comprises a needle receiving portion. The needle receiving portion in each jaw may have any convenient size or shape, and in some embodiments of the invention the needle receiving portion in each jaw comprises a channel. The channel may be an open or closed channel. The channel will be shaped appropriately for the dimensions of the needle.

In embodiments of the invention each lock comprises a resiliently deformable component.

In embodiments of the invention the resiliently deformable component comprises a spring. In such embodiments of the invention the spring may be adapted to bear down on the needle in order to lock the needle in a respective jaw.

In other embodiments of the invention each lock comprises a pressing element. The pressing element may be moveable within a respective jaw such that the pressing element bears down on the needle in order to lock the needle into the respective jaw.

The pressing element may be formed from a resiliently deformable material. In other embodiments, the pressing element is formed from a substantially rigid material.

In some embodiments of the invention, the thread receiving portion comprises an aperture formed between the first and second ends of the needle. In some embodiments of the invention the aperture is formed in a middle portion of the needle. The aperture may be formed completely within the thickness of the needle, or it may be formed so that it is open at one edge of the needle.

In embodiments of the invention where the aperture is open at one edge of the needle, the thread that will be used in the suturing process may be readily inserted into the aperture. Once the thread has been positioned within the aperture the needle may be closed around the aperture onto the thread so that the aperture closes around and holds the thread without leaving an external notch. Once the aperture has closed around the thread, the thread will be held in position by the walls of the needle.

In embodiments of the invention where the aperture is formed within the needle, the thread may be passed through the aperture. Once the thread is in position, the needle may be compressed such that the thickness of the needle in the area in which the aperture is formed, is reduced, and the needle is compressed onto the thread, thus holding the thread into position. Again, in this embodiment, the thread is held in position without requiring a notch to be formed on an external surface of the needle.

The absence of any external notch required to either hold the thread into position, or to drive the needle means that the suturing process is smoother.

An important feature of the sewing instrument according to embodiments of the invention is that it may be provided in a modular form, with the sewing device being readily attachable and detachable from the remainder of the sewing instrument.

The sewing instrument may comprise a shaft extending axially along axis the sewing instrument. The shaft may be rigid, or flexible. The shaft may be formed from separate shaft portions that are linked together or may be formed from a single tube or rod. There are many variations of the shaft that could be used to form a sewing instrument according to the invention, and the sewing device may be adapted to be readily attachable or detachable from any such shaft.

In embodiments of the invention, the first and second jaws comprise first and second crank mechanisms respectively, and the first drive mechanism comprises a slider moveable axially along the sewing instrument, a first slider crank linkage extending between the first crank mechanism and the slider, and a second crank mechanism extending between the second crank mechanism and the slider.

The slider may take any convenient form, and in some embodiments of the invention the slider comprises a disc.

In such embodiments of the invention, movement of the disc in an axial direction will cause movement of the first and second jaws between the open and closed positioned.

In embodiments of the invention, the first drive mechanism may comprise an elongate member operatively connected to the slider, the elongate members being moveable axially along the sewing instrument.

The elongate member may be contained within the shaft of the sewing instrument.

The elongate member may take any convenient form, and may for example comprise a tube extending from the proximal end of the instrument to the sewing device.

In other embodiments of the invention, the first drive mechanism further comprises a first tendon operatively connected to the slider. In such embodiments of the invention, the tendon may be used to apply a pulling force to the slider to cause movement of the slider from a first position to a second position towards the proximal end of the instrument. This courses movement of the first and second jaws to the open position.

In such embodiments of the invention, the first drive mechanism may further comprise a second tendon operatively connected to the slider, wherein when a pulling force is applied to the second tendon, the slider is caused to move towards the distal end of the sewing instrument and thus back to the first position, thus causing the jaws to move to the closed position.

In such embodiments of the invention, the first drive mechanism may further comprise a pulley, and the second tendon may be fixed to, and extend from the slider mechanism and around the pulley, and then to the proximal end of the instrument.

In some embodiments of the invention the sewing instrument further comprises a first bias for biasing the slider towards the first position. The first bias may comprise a spring, for example. In such embodiments, it is not necessary to have a second tendon to return the slider to the first position.

The second drive mechanism may comprise a rotary cam operatively engageable with the lock mechanism.

The second drive mechanism may further comprise a rotary shaft having a proximal end and distal end, the rotary cam being positioned at the distal end of the rotary shaft.

In such embodiments of the invention, rotation of the rotary shaft will cause the first and second locks to be either unlocked or locked respectively.

In some embodiments of the invention the first and second drive mechanisms comprise a combined drive mechanism.

In some embodiments of the invention the sewing instrument comprises an auto lock mechanism.

The invention will now be further described by way of example only with reference to the accompanying drawings in which:
Figure 1 is a schematic representation of a distal portion of a sewing instrument;
Figures 2 and 3 are schematic representations of a portion of the distal end of the sewing instrument of Figure 1 showing the sewing device in more detail with the first and second jaws in an open portion;
Figure 4 is a schematic representation showing the distal end of the instrument of Figure 1 showing the position of the jaws in the open position and showing the first lock in an unlocked position;
Figure 5 is a schematic representation of the distal end of the instrument of Figure 1 showing the needle in position and the first lock in a locked position;
Figure 6 is a schematic representation of the distal end of the instrument of Figure 1 showing the jaws in a closed position with the needle held by both jaws;
Figure 7 is a schematic representation showing the jaws still in a closed position but with the second lock in a locked position and the first lock in an open position;
Figure 8 is a schematic representation showing the jaws in an open position with the needle held unlocked in position in the second jaw;
Figure 9 is a schematic representation of an embodiment of a device according to aspects of the invention;
Figure 10 is a schematic representation of the instrument of Figure 9 showing a needle locking mechanism in more detail;
Figure 11 is a schematic representation of the instrument of Figure 9 showing the needle locked in the first jaw.
Figure 12 is a schematic representation of the instrument of Figure 9 showing the needle with both ends contained in respective jaw but with the lock of the second jaw in an unlocked position;
Figure 13 is a schematic representation of the instrument of Figure 9 with no needle in place;
Figure 14 is a schematic representation of a part of a sewing instrument;
Figure 15 is a schematic representation of a sewing device;
Figure 16 is a schematic representation of the sewing device of Figure 15 showing the internal parts in more detail;
Figure 17 is a schematic representation of the sewing device of Figure 15 showing the auto lock mechanism in more detail;
Figures 18 to 21 are schematic representations of parts of the auto locking mechanism shown in Figure 17;
Figures 22 and 23 are schematic representations showing the slider crank mechanism of an instrument as shown in Figure 15 coupled with a moving cam;
Figures 24 and 25 show the initial position of the components of the sewing device shown in Figure 15;
Figures 26 and 27 show a second stage in the working sequence of the dewing device;
Figure 28 and 29 show a position of the components in the third stage of the working sequence;
Figures 30 and 31 are schematic representations of a thread receiving portion formed in the needle of an embodiment of the invention;
Figures 32 and 33 are schematic representations of another embodiment of the invention having a different type of thread receiving portion.

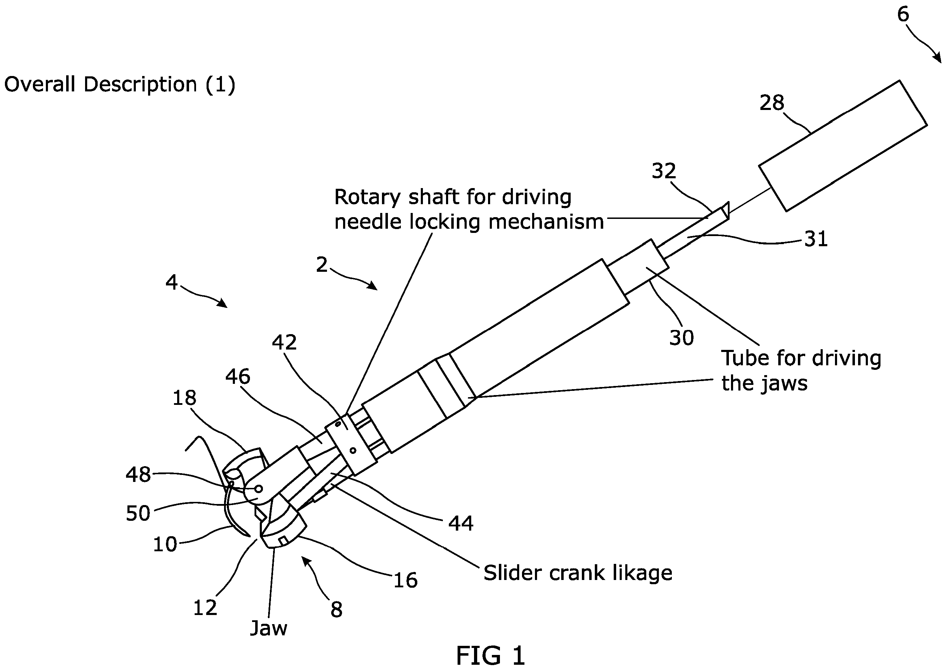

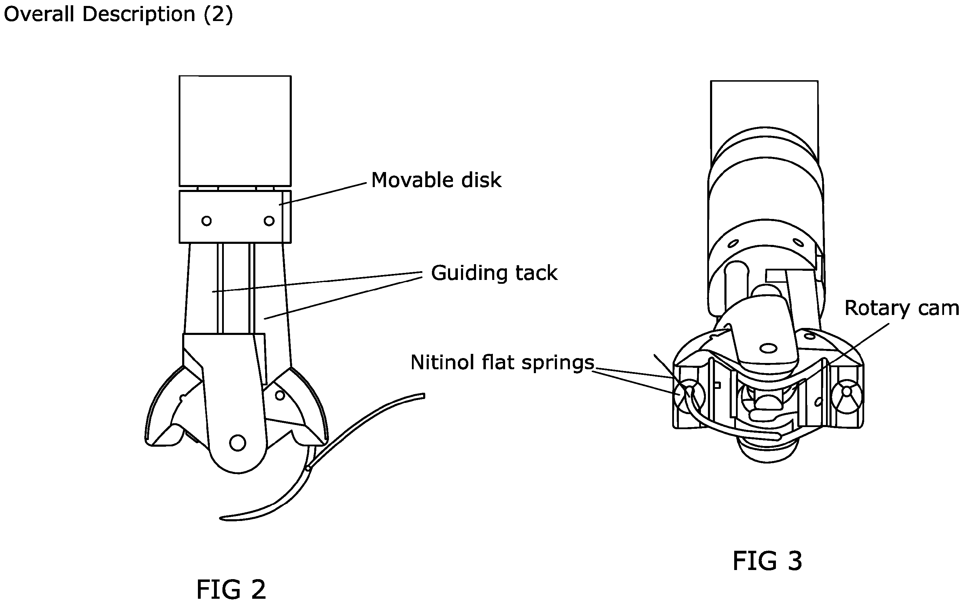

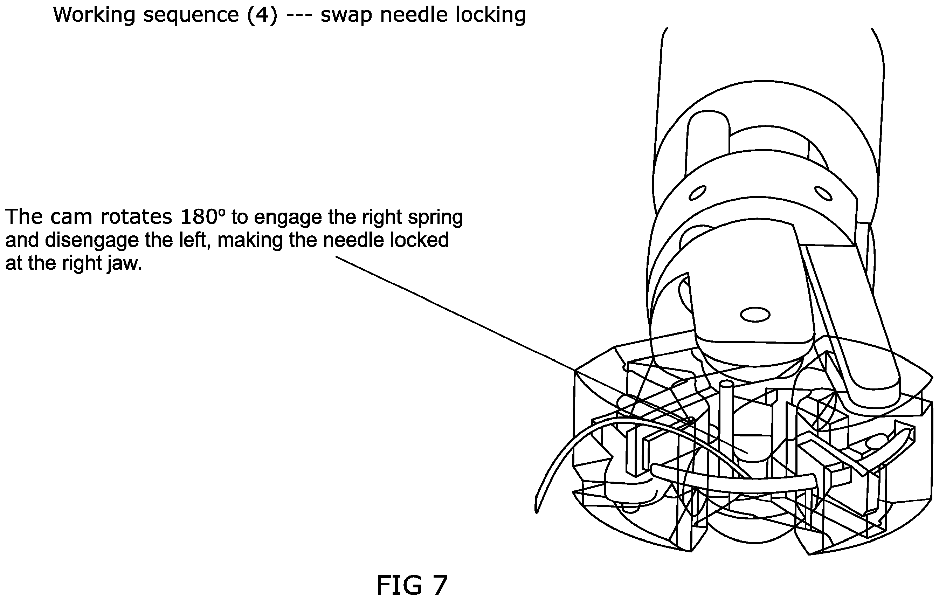

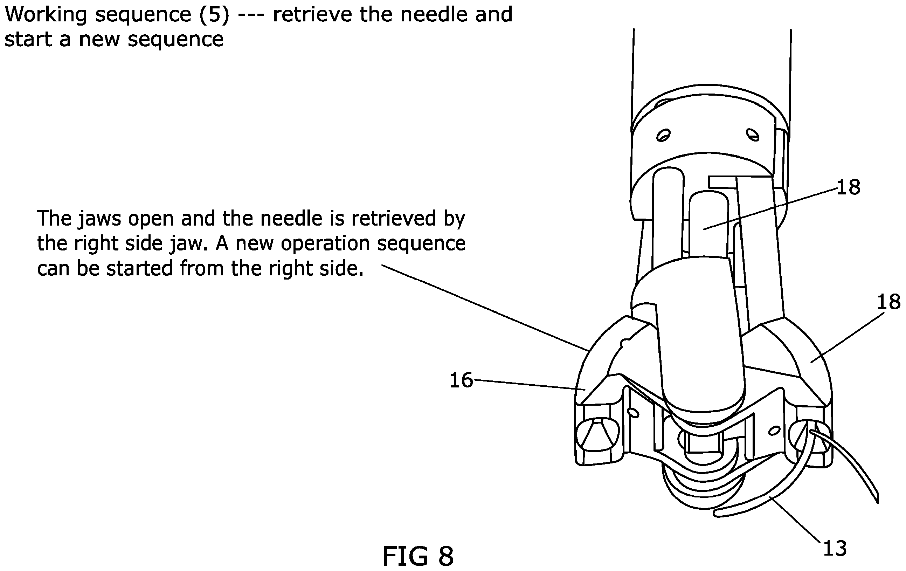

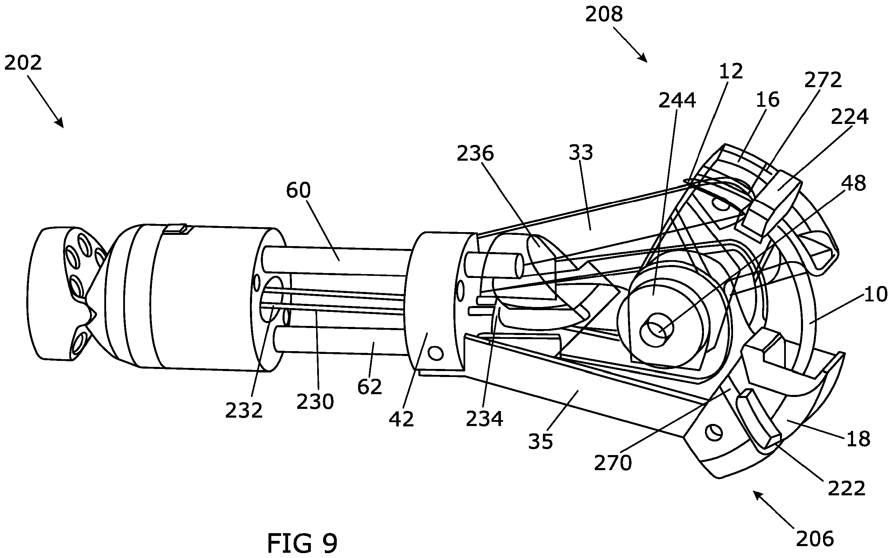

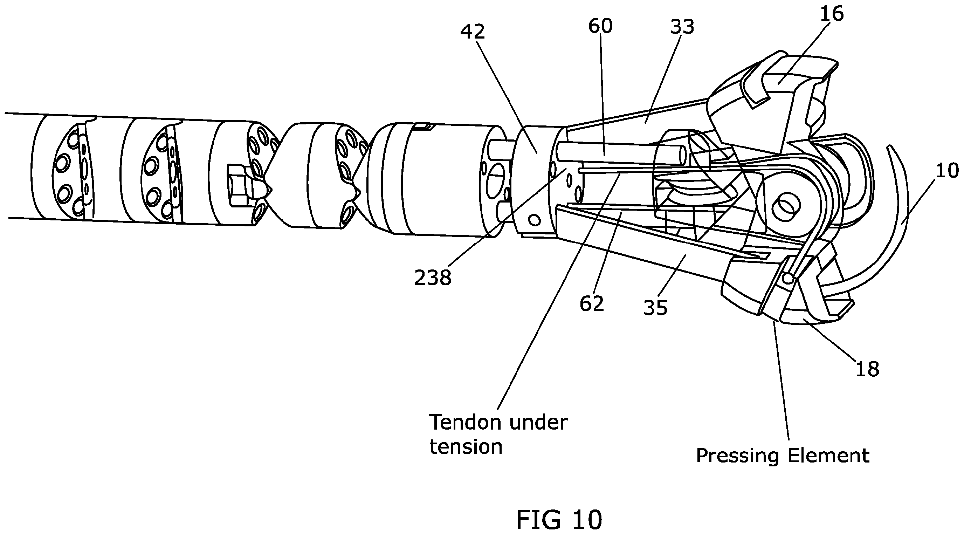

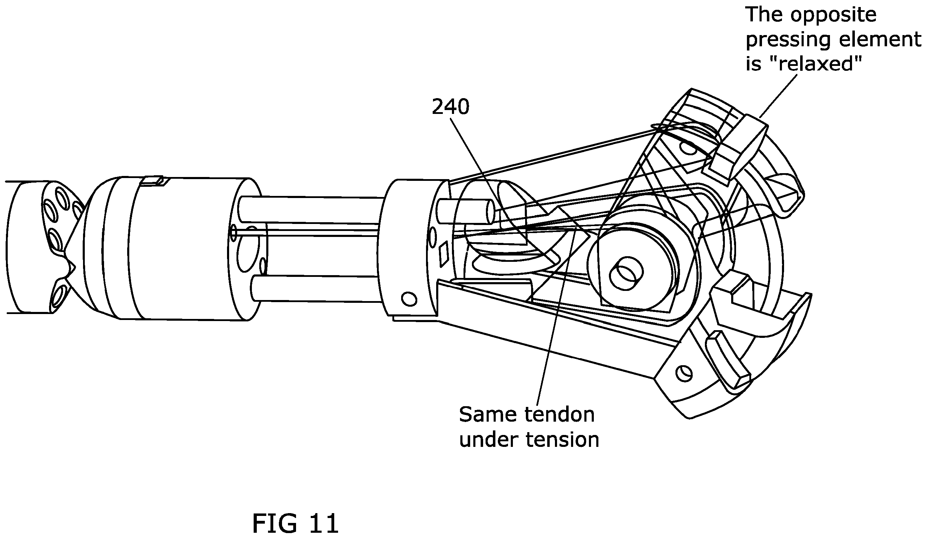

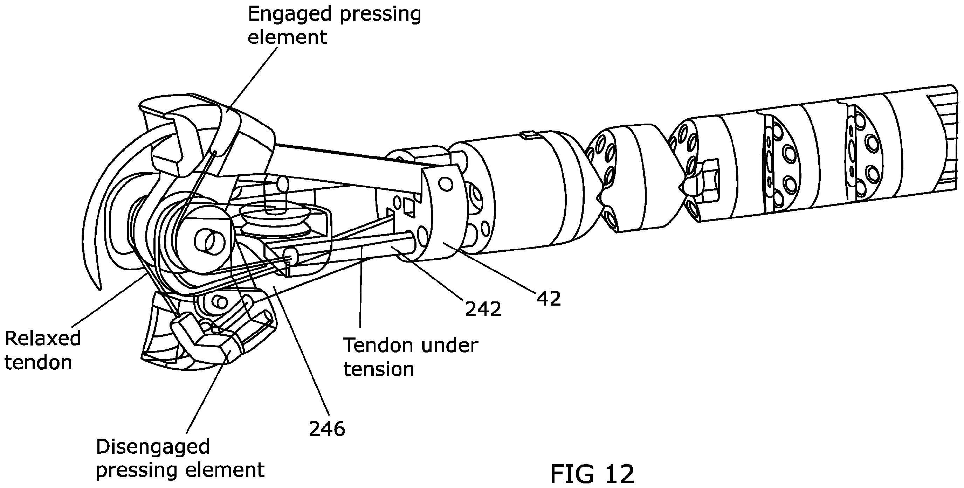

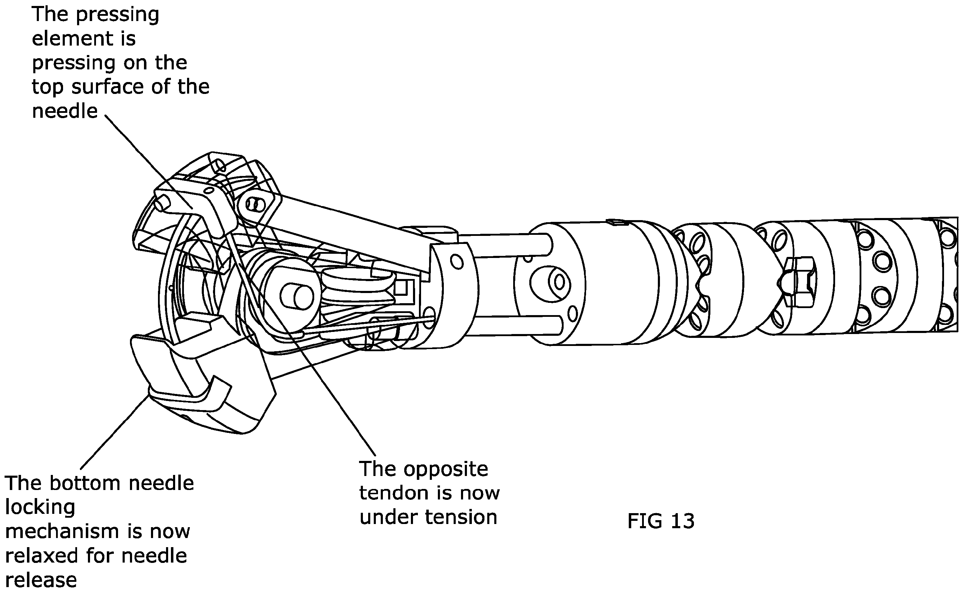

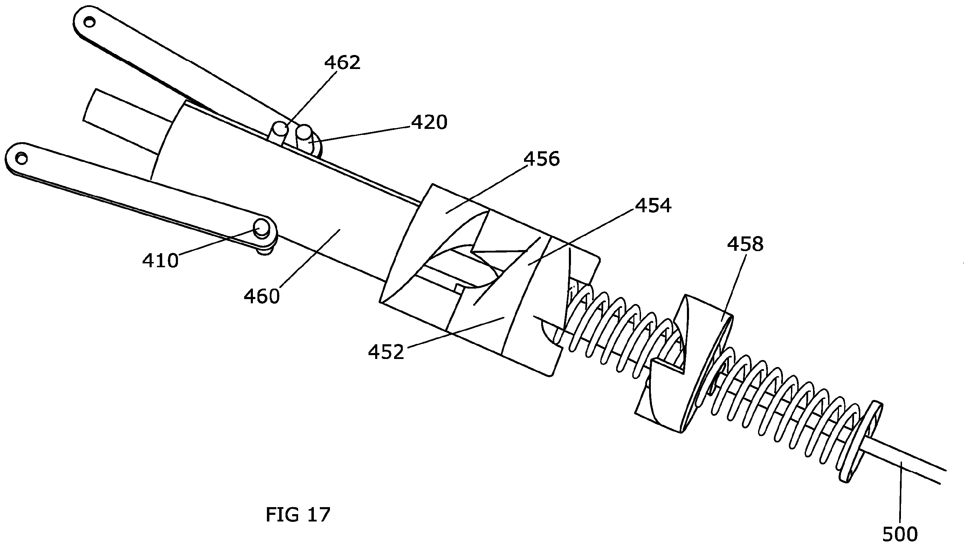

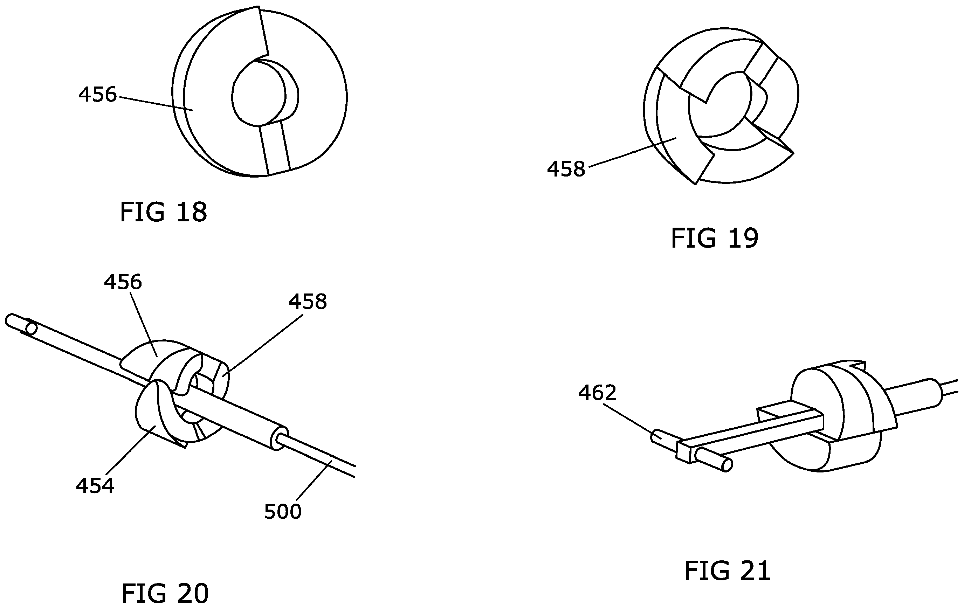

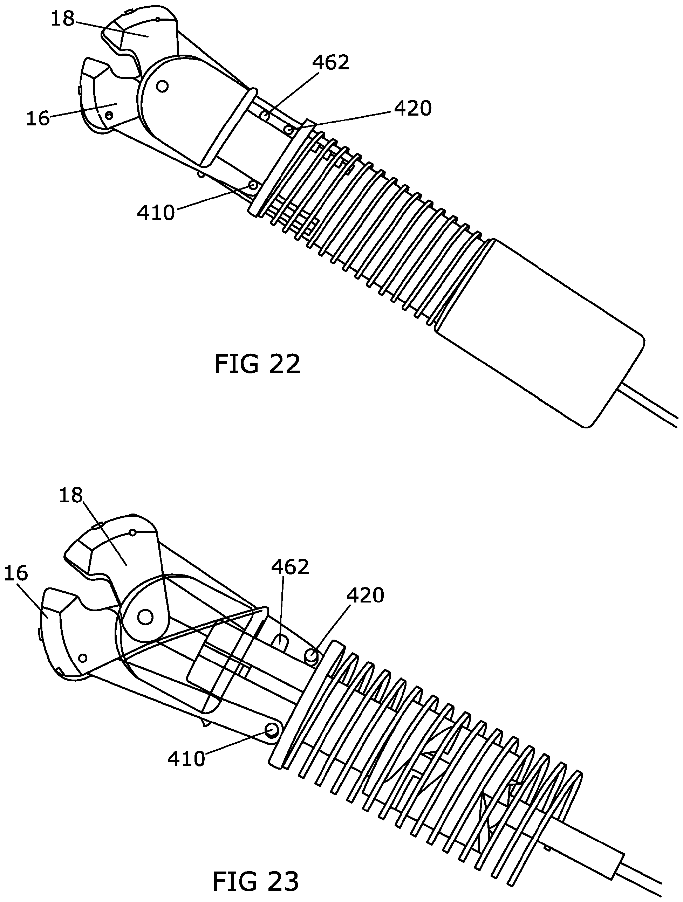

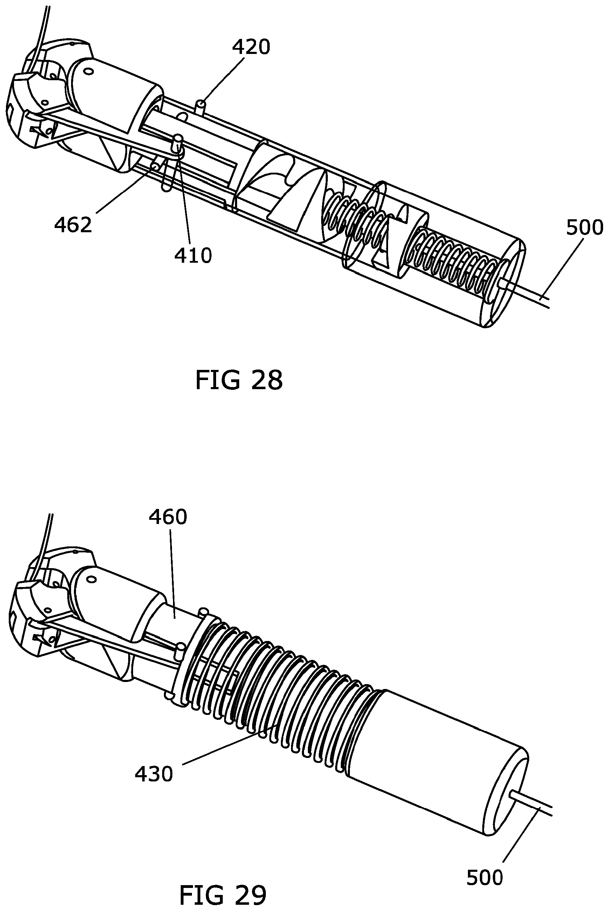

Embodiments of the invention are shown in Figures 9 to 13. The examples shown in Figures 1 to 8, 14a to 29 do not form part of the present invention.

Referring initially to Figure 1, a sewing instrument is designated generally by the reference number 2. The sewing instrument has a distal end 4 and a proximal end 6. The instrument 2 further comprises a sewing device 8 positioned at the distal end of the instrument. The sewing device comprises a needle 10 having a first point 12 at a first end and a second point 14 at a second end.

The needle is curved. The curve may be any suitable curve, but in this example the needle defines an arc extending over approximately 160 to 200 °.

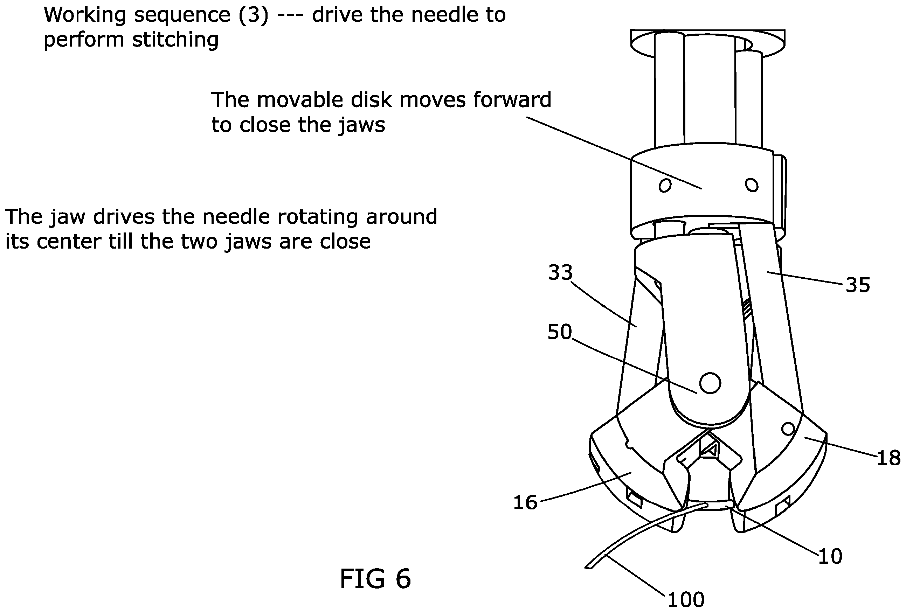

The sewing device 8 further comprises a first jaw 16 and a second jaw 18. The jaws 16, 18 are moveable between an open position as shown in Figure 1, and a closed position as shown in Figure 6. Movement of the jaws 16, 18 will be described in more detail below. The first jaw 16 is adapted to hold the needle 10 at the first end 13 and the second jaw 18 is adapted to hold the needle at the second end 15. In the example shown in Figure 1, the second end 15 is held in the second jaw 18, and the first end 13 is exposed. Each jaw 16, 18 comprises a working channel 38, 40 adapted to receive a respective end 13, 15 of the needle 10.

The sewing device 8 also comprises a lock mechanism. In this example the lock mechanism comprises a first lock and a second lock. The first and second locks are operatively connected to a rotary cam 26. The operation of the locking mechanism will be described in more detail below.

In this example the first and second locks each comprise a flat spring 34, 36. Each flat spring 34, 36 is designed with a slop which can facilitate the insertion of the needle.

When an end 13, 15 of the needle is inserted into a respective working channel 38, 40, the needle may be locked or clamped in position in a respective jaw by a force applied by the flat spring 34, 36 when the flat spring 34, 36 is moved to the locked position.

Each flat spring 34, 36 is operatively connected to the rotary cam 26. Rotation of the rotary cam 26 will cause one or other of the flat springs 34, 36 to move from an unlocked position to a locked position in which the respective flat spring bears down on the needle.

The sewing instrument 2 comprises a first drive mechanism 30 for driving the jaws 16, 18, and a second drive mechanism 32 for driving the locking mechanism.

The suturing instrument 2 further comprises an actuator 28 positioned at the proximal end 6 of the device. The actuator is operatively connected to the suturing device by a first drive mechanism 30 for driving the jaws 16, 18 and a second drive mechanism 32 for driving the locking mechanism.

The first drive mechanism 30 is, in this example in the form of a tube 31 extending axially along the shaft of the device 2 towards the distal end 4 of the device 2, a slider 42, and first and second slider crank linkages 33, 35. The tube is connected at a distal end to the slider 42, which in this embodiment is substantially disc shaped. The slider 42 is moveable axially along the device 2 along guides 60, 62, and is connected to the first and second slider crank linkages 33, 35. The jaws 16, 18 pivot between the open position as shown in Figure 1, and a closed positioned as shown in Figure 6.

The jaws 16, 18 pivot about pivot pin 48 which is arranged so that it extends through the centre of a circle an arc of which is defined by the needle 10.

The second drive mechanism 32 comprises a rotary shaft 37 running coaxially to the first drive mechanism 30, and rotary cam 26.

The rotary shaft 37 extends from the proximal end 6 of the instrument towards the distal end 4 of the device and is operatively connected to the rotary cam 26.

A working sequence of the suturing device will now be described.

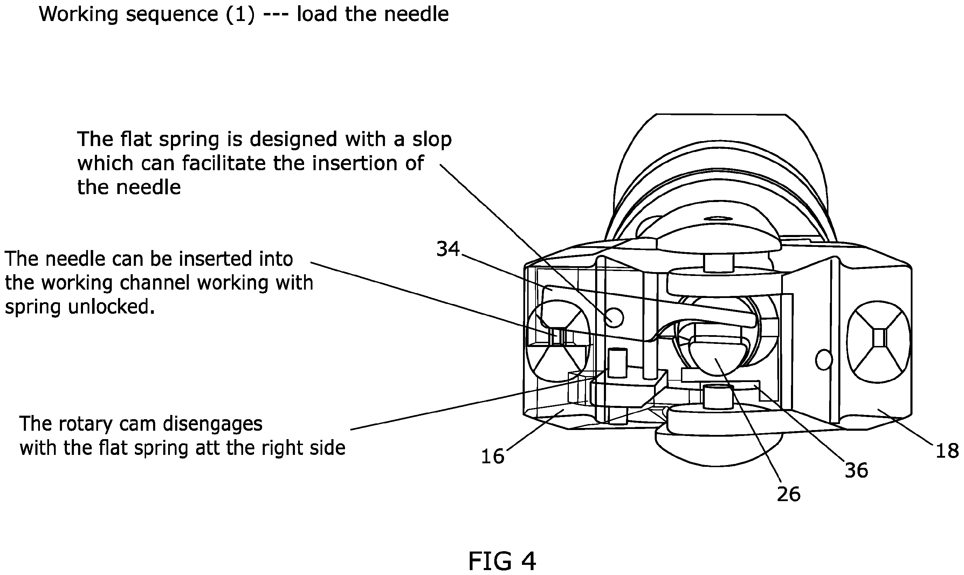

### Working Sequence 1 - load the needle (Figure 4)

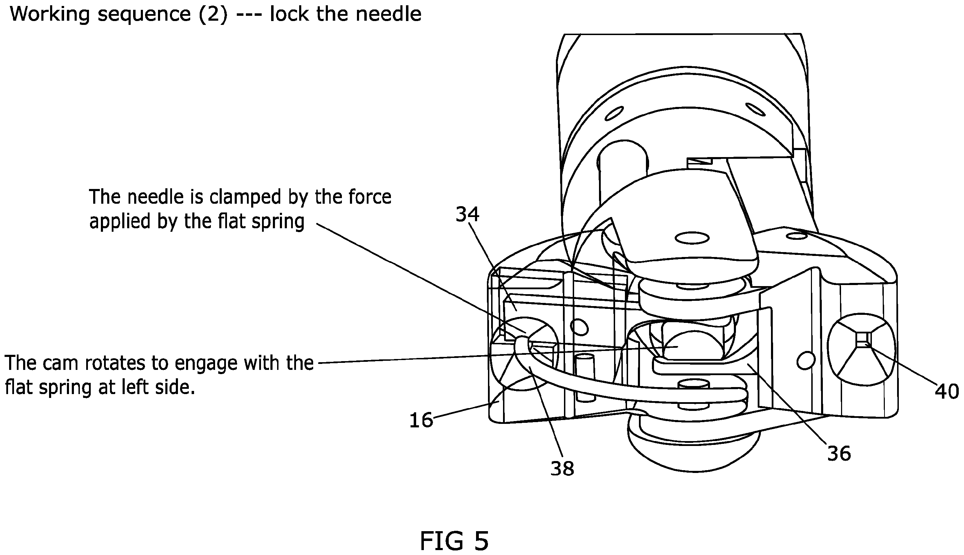

Initially, in order to load the needle, the jaws 16, 18 are in the open position. The first lock 34 is in an unlocked position, and the second lock 36 is in the locked position. This is because the rotary cam 26 is in the positioned shown in Figure 4 in which it is disengaged from the first lock 34 and engaged with the second lock 36. In this configuration, the first end 13 of the needle 10 (not shown in Figure 4) may be inserted into the first jaw 16 as shown in Figure 5. In this position, the second end 15 will be exposed.

### Working Sequence 2 - lock the needle (Figure 5)

Once the first end 13 of the needle 10 has been inserted into the first jaw 16, the rotary cam 26 is caused to rotate to the position shown in Figure 5 by rotating rotary shaft 37. In this position, the rotary cam 26 in is engages with the first lock 34. This causes the first lock 34 to bear down on the needle 10 which is positioned in working channel 38 thereby locking the needle in place in the first jaw 16.

### Working Sequence 3 - drive the needle to perform the stitching (Figure 6)

Next, the slider 42 is caused to move forward towards the jaws 16, 18 by causing the tube 31 to push on the slider 42. This causes the slider crank linkages 33, 35 to move the jaws 16, 18 to the closed positioned as shown in Figure 6. The jaws 16, 18 thus pivot about pivot pin 48 to move to the closed position. In this position the second end 15 of the needle 10 is within the working channel in jaw 18. This is the position in which the suturing device 2 can be inserted into a patient. In this position the needle is protected by the jaws 16, 18.

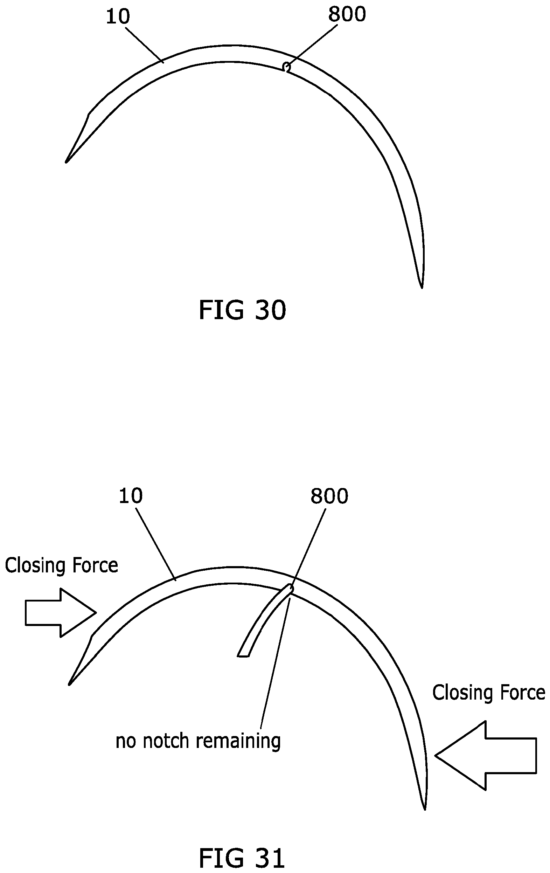

In the working sequence shown in Figure 6, thread 100 may then be attached to the needle 10 ready for suturing. The manner in which the thread 100 is held in the needle will be described in more detail below with reference to Figures 30 to 33.

### Working Sequence 4 - swap needle locking

The rotary cam is caused to rotate through 180° to engage with second lock 36 by rotating rotary shaft 37, and to engage with first lock 34. This means that the needle 10 is now locked by the second jaw 18.

### Working Sequence 5 - retrieve the needle and start a new sequence

The jaws 16, 18 are then caused to pivot to their open position by moving the tube 31 towards the proximal end of the instrument. This causes first end 13 to be exposed with the second end 15 being locked in jaw 18. A new sequence may now begin.

The tube 31 and rotary shaft 37 may be manually activated in order to drive the jaws 16, 18 and the locks 34, 36 as described here and above. Alternatively, the tube 31 and rotary shaft 37 may be caused to move by actuator 28 as is the case in the illustrated example. In such examples, operation of the sewing device may be automated. Referring now to Figures 9 to 13, a sewing instrument is designated generally by the reference numeral 202. Parts of the sewing instrument 202 that correspond to parts in the examples shown in Figures 1 to 8 have been given corresponding reference numerals for ease of reference.

In the embodiments shown in Figures 9 to 13, the lock mechanism comprises a first lock 222 and a second lock 224. Each of the locks comprises a pressing element which is positioned within a lock receiving portion 270, 272, in each of the jaws 16, 18. Movement of the jaws, 16, 18 is driven by first drive mechanism 230, which in this embodiment comprises a first tendon 232 which is fixably attached to slider 42 at one end thereof. An opposite end of the tendon 232 extends axially along the instrument 202 to the proximal end 6 of the instrument.

The first drive mechanism 230 further comprises a second tendon 234 which is also attached to the slider 42 at a first end thereof. The second tendon 234 extends around a pulley 236 also forming part of the first drive mechanism 230 then back through the slider 442 along the axis of the instrument 202 to the proximal end of the instrument 202. The second tendon 234 is attached to the slider 42 only at a first end thereof, and therefore when the tendon passes back through the slider 42 after extending around pulley 236, it is not attached to the slider 42.

In order to drive the jaws 16, 18, the first tendon 232 may be pulled either manually or automatically. This pulling action causes the slider 42 to move towards the proximal end of the instrument 202. This movement causes the slider crank linkages 33, 35 to move which in turn causes the jaws 16, 18 to move as described hereinabove with reference to the first embodiment of the invention.

In order to move the slider 42 towards the distal end of the instrument 202, the second tendon 234 may be pulled either manually or automatically. This movement causes the slider to move towards the distal end with consequential movement of the slider crank linkages 33, 35, with consequential movement of the jaw 16, 18.

Each of the locks 222, 224 is attached to a respective locking tendon 240, 242. Each tendon 240, 242 is attached to a respective lock 222, 224 at one end thereof. Each tendon extends around a lock pulley 244, 246 and through slider 42 to the proximal end of the instrument 202.

When it is required to lock the needle in one of the jaws 16, 18, a pulling action is applied to the appropriate tendon 240, 242. This pulling action pulls the respective pressing element down onto a respective end of the needle. At this same time, the other tendon 240, 242 is relaxed, thereby releasing the lock in the other jaw.

The locking tendons 240, 242 may be operated either manually or automatically in order to have a working sequence similar to that described hereinabove with reference to Figures 1 to 8.

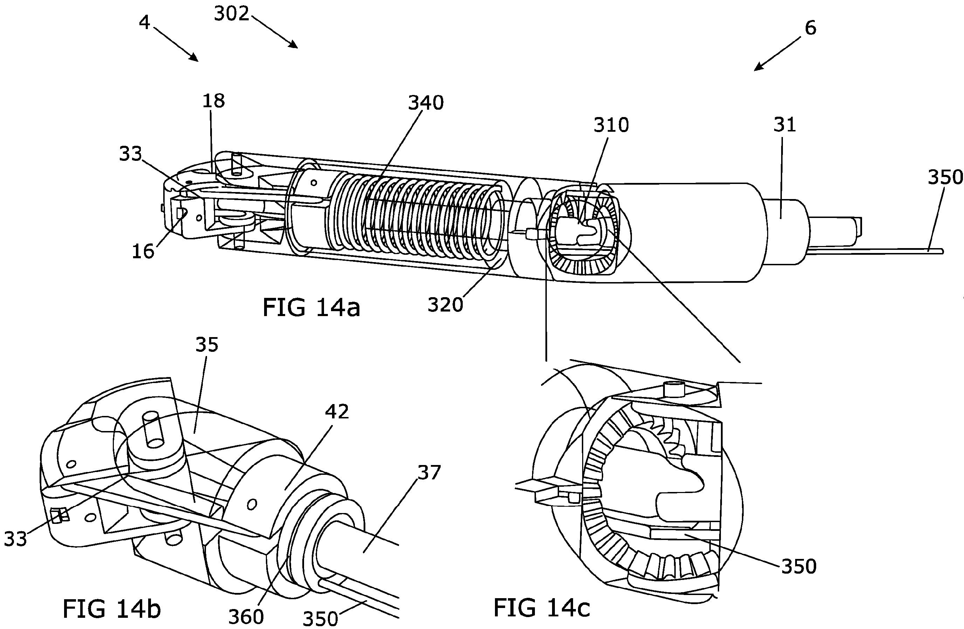

Turning now to Figures 14a, b and c, a sewing instrument is designated generally by the reference numeral 302.

The sewing instrument 302 is similar to the sewing instrument 2 described with reference to Figures 1 to 8, and parts of the sewing instrument 302 that correspond to parts of the sewing instrument 2 have been given corresponding reference numerals for ease of reference.

The sewing instrument 302 is articulated in that it has an elbow (hinge) joint 310 and a 360° rotational wrist (revolute) joint 320.

In this example, the first drive mechanism 330 comprises a bias in the form of a spring 340 which is adapted to provide a pushing actuation on the slide or crank linkages 33, 35. The first drive mechanism further comprises a tendon 350 which is connected to a rotational ring 360 which in turn is attached to the slider 42. The rotational ring prevents the tendon from twisting when the sewing device is rotated.

In order to move the jaw 16,18 from the closed position to the open position, a force is applied to the tendon 350 in order to move the slider towards the proximal end of the instrument.

When it is required to move the jaws to the closed position the tendon 350 is allowed to relaxed, and the spring 430 will return the jaw 16,18 to the closed position.

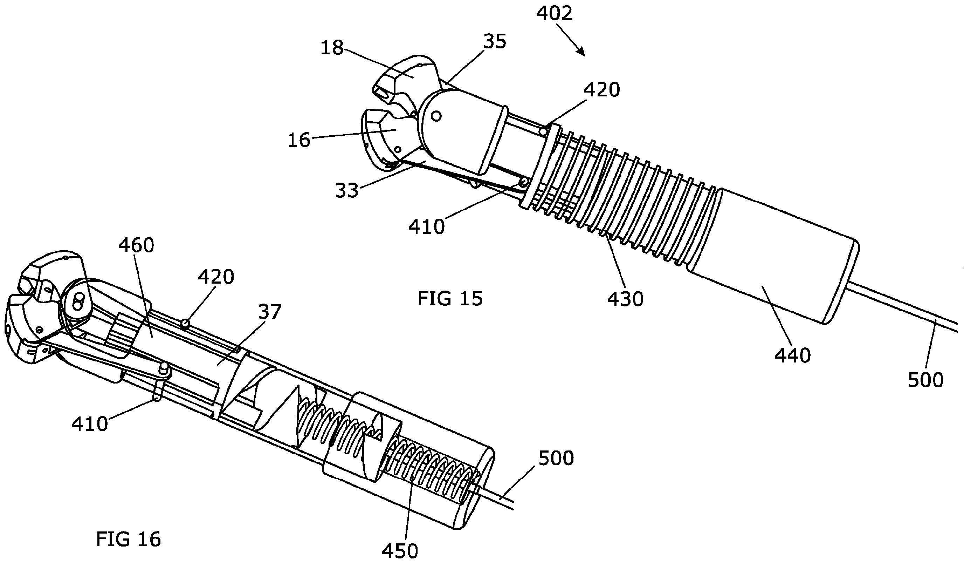

Figures 15 to 29 which illustrate a sewing instrument 402.

In this example, there is a combined drive mechanism replacing the separate first and second drive mechanisms in the previous embodiments, and an autolock mechanism.

The sewing instrument 402 comprises a sewing device having jaws 16, 18, slide crank linkages 33, 35 which linkages 33, 35 comprise pins 410, 420. The instrument 402 further comprises an outer spring 430. The outer spring 430 is held between the pins 410, 420, and a moveable outer portion 440 positioned towards the proximal end of the instrument 402.

Instrument 402 further comprises an inner spring 450, and a rotary shaft 37 for actuating the lock mechanism 20 via a rotary cam (not shown).

The instrument 402 further comprises a tendon 500 adapted to activate both the lock mechanism 20 and the jaw 16,18.

The inner spring 450 is operatively connected to a cam mechanism 452 which cam mechanism comprises a moving cam 454, a first fixed cam 456 and a second fixed cam 458. The instrument 402 also comprises a slider 460, and a slider pin 462 for coupling the slider 460 to the moving cam 454.

The autolocking mechanism causes the needle locking mechanism to automatically switch between the two jaws when the jaws 16, 18 are closed as will be explained below.

The tendon 500 is connected to the moving cam 454. When a user activates the device by pressing the moveable portion 440, the inner spring 450 pushes the moving cam towards the distal end of the instrument 402.

When the jaws 16, 18 are about to close, the moving cam 454 automatically rotates through a large angle, which in this example is 170° in order to switch the needle locking from one jaw to another.

When the jaws are opening, the moving cam 454 rotates a smaller angle which in this example is about 10°, in order to move to a preparatory trigger position for the next switching locking.

The moving cam 454 is adapted to rotate the rotary cam which forms part of the lock mechanism, during motion of the moving cam 454.

When the moving cam contacts the first fixed cam 456 the moving cam is forced to rotate through the large angle of around 170°. When the moving cam 454 contacts the second fixed cam 458, the moving cam is forced to rotate through a small angle of around 10° to be ready for the trigger position for the next sequence.

The small angle and the large angle together add up to 180°, so in each sequence the needle locking is switched from one jaw to another.

The needle locking switching is triggered when the jaw is fully closed otherwise it may fail in locking the needle. This means that it requires a delayed motion between the two movements.

The working sequence is set out below.

Initially, the needle is locked into one of the jaws 16, 18.

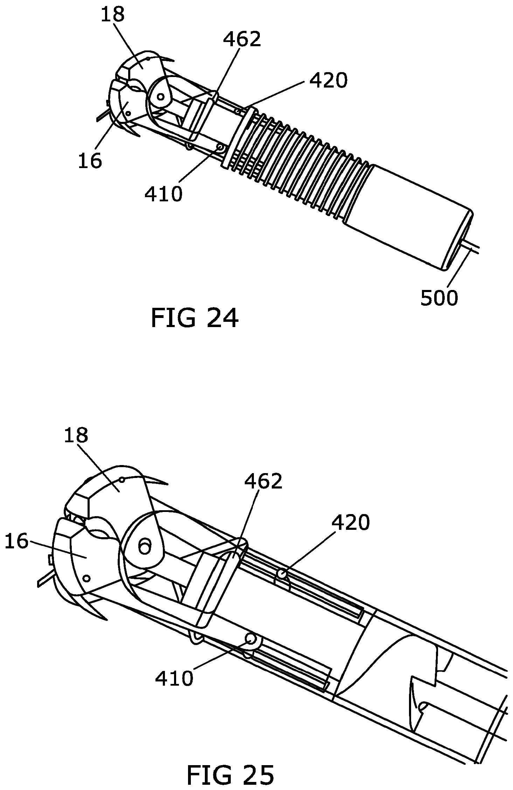

When the tendon 500 is pulled when it is connected to the moving cam 454, the slider pin 462 will move with the moving cam 454. When the pin 462 reaches the pins 410 and 420, the jaws 16, 18 will start to open. This is shown in Figures 24 and 25.

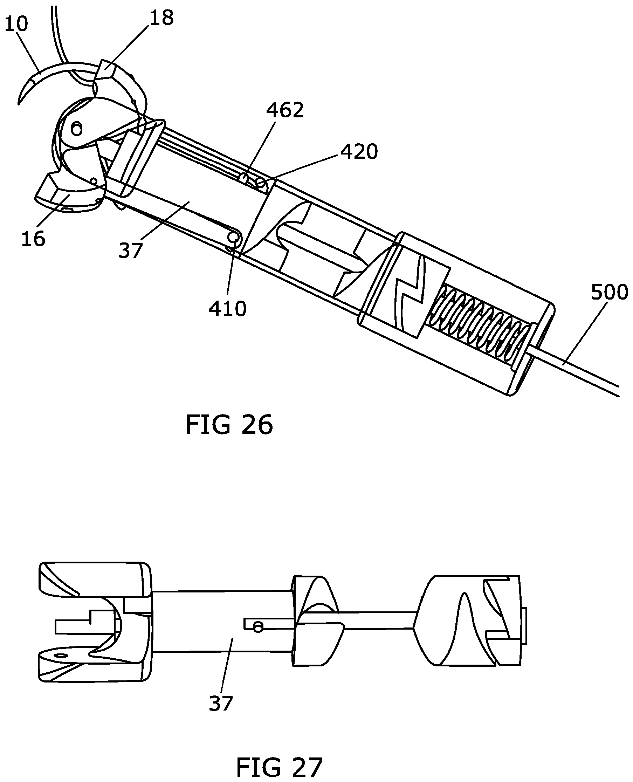

When the moving cam contacts with the second fixed 458 cam it rotates through the small angle of about 10° driving the moving cam 454 into a position where it can rotate through the larger angle of around 170° when engaging the first fixed cam 456 in the next sequence. The moving cam 454 drives a rotational shaft in order to lock the needle. This is shown in Figure 27.

When the moving cam contacts the first fixed cam 456, it rotates through the large angle of around 170°. At this point the jaws 16, 18 are forced to be closed by the outer spring. In each working sequence the rotary shaft rotates 180° so the needle is switched between the two jaws.

In all the embodiments described hereinabove, the needle comprises a thread receiving portion. This will now be described in more detail with reference to Figures 30 to 33.

As shown in Figures 30 and 31, the thread receiving portion 800 comprises an open aperture formed in an inner portion of the needle 10. The aperture is not fully closed. The thread may be placed within the aperture and the needle closed so that the hole closes around the thread without leaving the external notch.

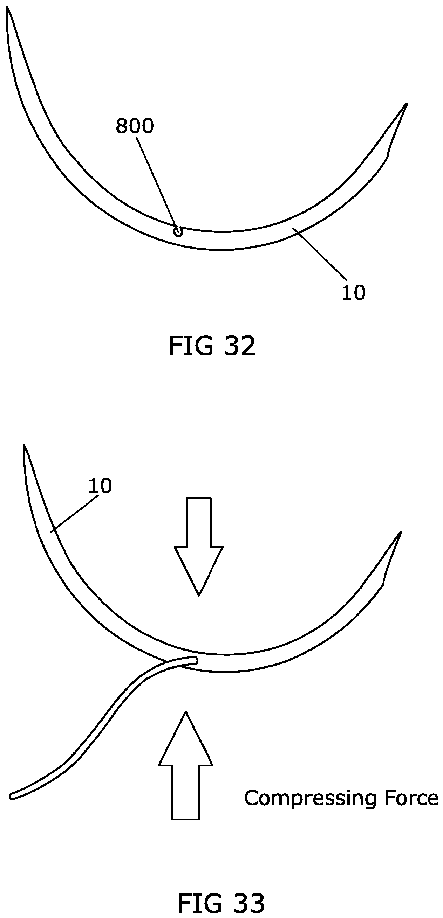

In another embodiment as shown in Figures 32 and 33, the aperture is completely enclosed within the needle 10. The thread may then be passed through the needle section. The needle is then compressed from above and below so that forces act on the inner and outer surfaces of the needle. This results in a compression of the needle onto the thread thus holding the thread in position.

## Claims

1. A sewing instrument (202) comprising:
a proximal end (6) and a distal end (4);
a longitudinal axis;
a sewing device (8) being positioned at the distal end of the sewing instrument, which sewing device comprises:
a curved needle (10) having a first end (13) and a second end (15), a first point at the first end and a second point at the second end, the needle further comprising a thread receiving portion (800);
a first jaw (16) and a second jaw (18), which jaws are moveable between an open position and a closed position, the first jaw being adapted to releasably hold the first end of the needle, and the second jaw being adapted to releasably hold the second end of the needle; and
a lock mechanism comprising a first lock (222) for locking the needle in the
first jaw, and a second lock (224) for locking the needle in the second jaw;
the sewing instrument further comprising:
a first drive mechanism (230) for moving the jaws between the open position and the closed position; and
a second drive mechanism for operating the first and second locks,
**characterised in that** the second drive mechanism comprises a first lock tendon (240) attachable to the first lock, and a second lock tendon attachable (242) to the second lock.

2. A sewing instrument (202) as claimed in claim 1 wherein the curved needle (10) substantially defines an arc of a circle.

3. A sewing instrument (202) as claimed in claim 1 or claim 2 wherein the sewing device (8) further comprises a pivot pin (48), the first and second jaws (16, 18) being pivotable about the pivot pin.

4. A sewing instrument (202) as claimed in claim 3 wherein the pivot pin (48) is positioned substantially at the centre of a circle, an arc of which is defined by the needle (10).

5. A sewing instrument (202) as claimed in any one of the preceding claims wherein each lock (222, 224) comprises a resiliently deformable component.

6. A sewing instrument (202) as claimed in any one of the preceding claims wherein each lock (222, 224) comprises a pressing element.

7. A sewing instrument (202) as claimed in any one of the preceding claims wherein the thread receiving portion (800) comprises an aperture formed between the first and second ends (13, 15) of the needle (10) and, optionally, in the middle portion of the needle.

8. A sewing instrument (202) as claimed in any one of the preceding claims comprising a shaft (37) extending axially along the sewing instrument.

9. A sewing instrument (202) as claimed in any one of the preceding claims wherein the first and second jaws (16, 18) comprise first and second crank mechanisms respectively, and the first drive mechanism comprises a slider (42) moveable axially along the sewing instrument, a first slider crank linkage (33) extending between the first crank mechanism and the slider, and a second crank linkage (35) extending between the second crank mechanism and the slider.

10. A sewing instrument (202) as claimed in claim 9 wherein the first drive mechanism comprises an elongate member (31) operatively connected to the slider (42) which elongate member is moveable axially along the sewing instrument.

11. A sewing instrument (202) as claimed in claim 9 wherein the first drive mechanism comprises a first tendon (232) operatively connected to the slider (42).

12. A sewing instrument (202) as claimed in claim 11 wherein the first drive mechanism further comprises a second tendon (234) operatively connected to the slider (42).

13. A sewing instrument (202) as claimed in any one of the preceding claims further comprising a first bias for biasing the slider (42) towards a first position.

14. A sewing instrument (202) as claimed in any one of claims 1 to 8 wherein the first and second drive mechanisms (230) comprise a combined drive mechanism.

15. A sewing instrument (202) as claimed in any one of the preceding claims comprising an auto lock mechanism.

## Patentansprüche

1. Nähinstrument (202), das Folgendes umfasst:
ein proximales Ende (6) und ein distales Ende (4);
eine Längsachse;
eine Nähvorrichtung (8), die an dem distalen Ende des Nähinstruments positioniert ist, wobei die Nähvorrichtung Folgendes umfasst:
eine gebogene Nadel (10), die ein erstes Ende (13) und ein zweites Ende (15), eine erste Spitze an dem ersten Ende und eine zweite Spitze an dem zweiten Ende aufweist, wobei die Nadel ferner einen Fadenaufnahmeabschnitt (800) umfasst;
eine erste Backe (16) und eine zweite Backe (18), wobei die Backen zwischen einer offenen Position und einer geschlossenen Position bewegbar sind, wobei die erste Backe angepasst ist, um das erste Ende der Nadel lösbar zu halten, und die zweite Backe angepasst ist, um das zweite Ende der Nadel lösbar zu halten; und
einen Verriegelungsmechanismus, der eine erste Verriegelung (222) zum Verriegeln der Nadel in der ersten Backe und eine zweite Verriegelung (224) zum
Verriegeln der Nadel in der zweiten Backe umfasst;
wobei das Nähinstrument ferner Folgendes umfasst:
einen ersten Antriebsmechanismus (230) zum Bewegen der Backen zwischen der offenen Position und der geschlossenen Position; und
einen zweiten Antriebsmechanismus zum Betätigen der ersten und der zweiten Verriegelung,
**dadurch gekennzeichnet, dass** der zweite Antriebsmechanismus einen ersten Verriegelungsspanndraht (240), der an der ersten Verriegelung befestigbar ist, und einen zweite Verriegelungsspanndraht (242) umfasst, der an der zweiten Verriegelung befestigbar ist.

2. Nähinstrument (202) nach Anspruch 1, wobei die gebogene Nadel (10) im Wesentlichen einen Kreisbogen definiert.

3. Nähinstrument (202) nach Anspruch 1 oder 2, wobei die Nähvorrichtung (8) ferner einen Bolzen (48) umfasst, wobei die erste und die zweite Backe (16, 18) um den Bolzen herum schwenkbar sind.

4. Nähinstrument (202) nach Anspruch 3, wobei der Bolzen (48) im Wesentlichen in der Mitte eines Kreises positioniert ist, dessen Bogen durch die Nadel (10) definiert ist.

5. Nähinstrument (202) nach einem der vorhergehenden Ansprüche, wobei jede Verriegelung (222, 224) eine elastisch verformbare Komponente umfasst.

6. Nähinstrument (202) nach einem der vorhergehenden Ansprüche, wobei jede Verriegelung (222, 224) ein Druckelement umfasst.

7. Nähinstrument (202) nach einem der vorhergehenden Ansprüche, wobei der Fadenaufnahmeabschnitt (800) eine Öffnung umfasst, die zwischen dem ersten und dem zweiten Ende (13, 15) der Nadel (10) und optional in dem Mittelabschnitt der Nadel ausgebildet ist.

8. Nähinstrument (202) nach einem der vorhergehenden Ansprüche, das einen Schaft (37) umfasst, der sich entlang des Nähinstruments axial erstreckt.

9. Nähinstrument (202) nach einem der vorhergehenden Ansprüche, wobei die erste und die zweite Backe (16, 18) jeweils einen ersten und einen zweiten Kurbelmechanismus umfassen und der erste Antriebsmechanismus einen Schieber (42), der entlang des Nähinstruments axial bewegbar ist, ein erstes Schubkurbelgetriebe (33), das sich zwischen dem ersten Kurbelmechanismus und dem Schieber erstreckt, und ein zweites Kurbelgetriebe (35) umfasst, das sich zwischen dem zweiten Kurbelmechanismus und dem Schieber erstreckt.

10. Nähinstrument (202) nach Anspruch 9, wobei der erste Antriebsmechanismus ein längliches Bauteil (31) umfasst, das mit dem Schieber (42) wirkverbunden ist, wobei das längliche Bauteil entlang des Nähinstruments axial bewegbar ist.

11. Nähinstrument (202) nach Anspruch 9, wobei der erste Antriebsmechanismus einen ersten Spanndraht (232) umfasst, der mit dem Schieber (42) wirkverbunden ist.

12. Nähinstrument (202) nach Anspruch 11, wobei der erste Antriebsmechanismus ferner einen zweiten Spanndraht (234) umfasst, der mit dem Schieber (42) wirkverbunden ist.

13. Nähinstrument (202) nach einem der vorhergehenden Ansprüche, das ferner eine erste Vorspannung zum Vorspannen des Schiebers (42) in Richtung einer ersten Position umfasst.

14. Nähinstrument (202) nach einem der Ansprüche 1 bis 8, wobei der erste und der zweite Antriebsmechanismus (230) einen kombinierten Antriebsmechanismus umfassen.

15. Nähinstrument (202) nach einem der vorhergehenden Ansprüche, das einen automatischen Verriegelungsmechanismus umfasst.

## Revendications

1. Instrument de couture (202) comprenant :
une extrémité proximale (6) et une extrémité distale (4) ;
un axe longitudinal ;
un dispositif de couture (8) étant positionné à l'extrémité distale de l'instrument de couture, lequel dispositif de couture comprend :
une aiguille incurvée (10) ayant une première extrémité (13) et une seconde extrémité (15), un premier point à la première extrémité et un seconde point à la seconde extrémité, l'aiguille comprenant en outre une partie de réception de fil (800) ;
une première mâchoire (16) et une seconde mâchoire (18), lesquelles mâchoires sont mobiles entre une position ouverte et une position fermée, la première mâchoire étant adaptée pour maintenir de manière amovible la première extrémité de l'aiguille, et la seconde mâchoire étant adaptée pour maintenir la seconde extrémité de l'aiguille ; et
un mécanisme de verrouillage comprenant un premier verrou (222) servant à verrouiller l'aiguille dans la première mâchoire, et un second verrou (224) servant à verrouiller l'aiguille dans la seconde mâchoire ;
l'instrument de couture comprenant en outre :
un premier mécanisme d'entraînement (230) servant à déplacer les mâchoires entre la position ouverte et la position fermée ; et
un second mécanisme d'entraînement servant à actionner les premier et second verrous,
**caractérisé en ce que** le second mécanisme d'entraînement comprend un premier tendon de verrouillage (240) pouvant être fixé au premier verrou, et un second tendon de verrouillage pouvant être fixé (242) au second verrou.

2. Instrument de couture (202) selon la revendication 1, dans lequel l'aiguille incurvée (10) définit sensiblement un arc de cercle.

3. Instrument de couture (202) selon la revendication 1 ou la revendication 2, dans lequel le dispositif de couture (8) comprend en outre un axe d'articulation (48), les première et seconde mâchoires (16, 18) pouvant pivoter autour de l'axe d'articulation.

4. Instrument de couture (202) selon la revendication 3, dans lequel l'axe d'articulation (48) est positionné sensiblement au centre d'un cercle, dont un arc est défini par l'aiguille (10).

5. Instrument de couture (202) selon l'une quelconque des revendications précédentes, dans lequel chaque verrou (222, 224) comprend un composant déformable élastique.

6. Instrument de couture (202) selon l'une quelconque des revendications précédentes, dans lequel chaque verrou (222, 224) comprend un élément de pression.

7. Instrument de couture (202) selon l'une quelconque des revendications précédentes, dans lequel la partie de réception de fil (800) comprend une ouverture formée entre les première et seconde extrémités (13, 15) de l'aiguille (10) et, éventuellement, dans le partie médiane de l'aiguille.

8. Instrument de couture (202) selon l'une quelconque des revendications précédentes, comprenant un arbre (37) s'étendant de manière axiale le long de l'instrument de couture.

9. Instrument de couture (202) selon l'une quelconque des revendications précédentes, dans lequel les première et seconde mâchoires (16, 18) comprennent respectivement des premier et second mécanismes à manivelle, et le premier mécanisme d'entraînement comprend un curseur (42) mobile de manière axiale le long de l'instrument de couture, une première tringlerie de manivelle de curseur (33) s'étendant entre le premier mécanisme de manivelle et le curseur, et une seconde tringlerie de manivelle (35) s'étendant entre le second mécanisme de manivelle et le curseur.

10. Instrument de couture (202) selon la revendication 9, dans lequel le premier mécanisme d'entraînement comprend un élément allongé (31) de manière fonctionnelle relié au curseur (42) lequel élément allongé est mobile de manière axiale le long de l'instrument de couture.

11. Instrument de couture (202) selon la revendication 9, dans lequel le premier mécanisme d'entraînement comprend une première armature (232) reliée de manière fonctionnelle au curseur (42).

12. Instrument de couture (202) selon la revendication 11, dans lequel le premier mécanisme d'entraînement comprend en outre une seconde armature (234) reliée de manière fonctionnelle au curseur (42).

13. Instrument de couture (202) selon l'une quelconque des revendications précédentes, comprenant en outre une première sollicitation destinée à solliciter le curseur (42) vers une première position.

14. Instrument de couture (202) selon l'une quelconque des revendications 1 à 8, dans lequel les premier et second mécanismes d'entraînement (230) comprennent un mécanisme d'entraînement combiné.

15. Instrument de couture (202) selon l'une quelconque des revendications précédentes, comprenant un mécanisme de verrouillage automatique.
